# EUROPÄISCHE PATENTSCHRIFT

(11) **EP 1 931 404 B1**
(45) Veröffentlichungstag und Bekanntmachung des Hinweises auf die Patenterteilung: **03.08.2011**
(21) Anmeldenummer: 06840916.8
(22) Anmeldetag: 02.09.2006
(51) Int. Cl.: A61M 1/34, A61M 1/02, B01D 37/00

(54) **VERFAHREN ZUR ENTFERNUNG VON LEUKOZYTEN AUS BLUT**
METHOD FOR THE ELIMINATION OF LEUKOCYTES FROM BLOOD
PROCEDE D'EXTRACTION DE LEUCOCYTES A PARTIR DE SANG

(30) Priorität: 09.09.2005 DE 102005043237; 22.08.2006 DE 102006039197
(43) Veröffentlichungstag der Anmeldung: 18.06.2008
(73) Patentinhaber: Membrana GmbH, 42289 Wuppertal (DE)
(72) Erfinder: LEMKE, Horst-Dieter, 63785 Obernburg (DE); WIESE, Frank, 42389 Wuppertal (DE); VON HARTEN, Bodo, 42119 Wuppertal (DE); KÖNIG, Martin, 40225 Düsseldorf (DE); RAMLOW, Wolfgang, 18059 Rostock (DE)
(74) Vertreter: Schröder, Richard
(86) Internationale Anmeldenummer: PCT/EP2006/008585
(87) Internationale Veröffentlichungsnummer: WO 2007/057065

(56) Entgegenhaltungen:
- EP-A1- 0 312 595
- WO-A-95/18665
- US-A- 4 925 572
- US-B1- 6 193 896
- BRUISTEN S M ET AL: "EFFICIENCY OF WHITE CELL FILTRATION AND A FREEZE-THAW PROCEDURE FOR REMOVAL OF HIV-INFECTED CELLS FROM BLOOD" TRANSFUSION, AMERICAN ASSOCIATION OF BLOOD BANKS, BETHESDA, MD, US, Bd. 30, November 1990 (1990-11), Seiten 833-837, XP002065086 ISSN: 0041-1132
- BONTADINI A ET AL: "Comparative analysis of six different white cell-reduction filters for packed red cells" TRANSFUSION (BETHESDA), Bd. 34, Nr. 6, 1994, Seiten 531-535, XP009078313 ISSN: 0041-1132
- KAPLAN A A ET AL: "COMPLEMENT KINETICS DURING CONTINUOUS ARTERIOVENOUS HEMOFILTRATION STUDIES WITH A NEW POLYSULFONE HEMOFILTER" BLOOD PURIFICATION, Bd. 6, Nr. 1, 1988, Seiten 27-36, XP009078393 ISSN: 0253-5068

## Beschreibung

Die Erfindung betrifft ein Verfahren zur Entfernung von Leukozyten aus Blut sowie eine Vorrichtung zur Durchführung dieses Verfahrens.

Blut besteht im wesentlichen aus Plasma und zellulären Elementen. Dazu zählen Erythrocyten (rote Blutkörperchen), Thrombozyten (Blutplättchen, Platelets) und Leukocyten (weiße Blutkörperchen). Zu den weißen Blutkörperchen zählen Lymphocyten, Monocyten und neutrophile Granulocyten (Neutrophile, PMN). Lymphocyten spielen eine entscheidende Rolle bei der spezifischen Immunität, Monocyten und neutrophile Granulozyten sind an der unspezifischen Immunabwehr oder Entzündungsreaktion beteiligte Zelltypen. Ihre Aufgabe ist es z.B., eingedrungene Mikroorganismen zu vernichten, die vorher von bestimmten körpereigenen Proteinen (etwa von C3b des Komplementsystems oder von Immunglobulin IgG) entsprechend als fremd markiert worden sind.

Haben die Zellen sich den eingedrungenen Mikroorganismen genähert, schütten sie sowohl Sauerstoffradikale als auch Proteasen aus, durch welche die Mikroorganismen abgetötet werden um danach phagozytiert zu werden. Wenn diese Reaktion nicht komplett abläuft oder außer Kontrolle gerät und chronisch wird, kann durch Freisetzung der agressiven Sauerstoffradikale und Proteasen auch körpereigenes Gewebe geschädigt werden. Während der Entzündung kommt es zwischen allen Zelltypen zu einer intensiven Kommunikation und Koordination, die sich u.a. verschiedener Cytokine bedient. Die Reaktion ist sehr komplex und noch nicht vollständig aufgeklärt. Sie führt aber letztlich zu den klinisch zu beobachtenden Symptomen der Entzündung von Schwellung, Rötung und Fieber. Charakteristisch ist u.a. ein Anstieg von im Knochenmark gebildeten und im Blut zirkulierenden Monocyten und neutrophilen Granulocyten und von bestimmten Botenstoffen. Neben den schon genannten proinflammatorischen Cytokinen werden auch die Komplementproteine C3a und C5a gebildet, die durch Aktivierung der Proteine C3 und C5 entstehen und die als Maß der Komplementaktivierung während der Entzündungs- oder Akutphasereaktion dienen.

Bei einer Reihe von entzündlichen Erkrankungen werden heute extrakorporale Therapien eingesetzt, etwa bei der Collitis ulcerosa, dem Morbus Crohn und der rheumatoiden Arthritis. Nach dem gegenwärtigen Stand der Technik werden dafür dem Patienten eine gewisse Anzahl von Zellen (wahrscheinlich speziell Monocyten und neutrophile Granulocyten) entfernt, indem das Blut des Patienten extrakorporal rezirkuliert und mit einem Zellfilter behandelt wird. Beispielsweise werden Säulen, die mit Partikeln, bzw. Beads aus Zelluloseacetat gefüllt sind, als Leucocytenfilter verwendet. Hier erfolgt die Entfernung hauptsächlich über Zelladsorption von Zellen an der Oberfläche der Beads. Solche Produkte sind bereits kommerziell erhältlich. Hier wird Blut durch eine Säule, die Zelluloseazetat Kugeln enthält, geleitet. Die Zelluloseazetat-Kugeln reduzieren speziell die im Blut enthaltenen Granulozyten und Monozyten durch Adsorption.

US 6,498,007 offenbart ein Verfahren zur Entfernung von Leukozyten aus Blut durch Adsorption an einen Träger. Dabei wird Blut mit diesem Träger in Kontakt gebracht, vorzugsweise in Form von sog. Beads, wobei der Träger eine höhere Affinität zu infizierten, aktivierten oder defekten Leukozyten aufweist als zu nicht infizierten, aktivierten bzw. defekten Leukozyten.

Alternativ werden auch Vliese oder Gewebe für die extrakorporale Entfernung von Leukozytenfilter eingesetzt. Beispielsweise werden für die Entfernung von Leukocyten aus einer Blutkonserve für die Transfusion (etwa eines Erythrocyten-oder eines Plateletkonzentrats) Produkte auf der Basis von Vliesen verwendet, bei denen die Zellentfernung überwiegend über mechanische Filtration mittels des Vlieses stattfindet. Für die Transfusion werden typischerweise Batches von 500 ml Blut in weniger als einer halben Stunde gefiltert. Der Prozeß läuft gravitationsbetrieben im single pass und nicht im Kreislauf ab. Damit ein Zellfilter im extrakorporalen Kreislauf eingesetzt werden kann, müssen etwa 1-61 Blut pumpengetrieben für einige Stunden gefiltert werden können. Dafür sind Polypropylenvliese in einem zylindrischen Gehäuse mit einem zuführenden Anschluss an der Stirnseite und einem abführenden Anschluss an der gegenüberliegenden Stirnseite kommerziell erhältlich. Mit dem dort verwendeten Vlies werden Leukozyten aufgrund von Filtrations- und Adsorptionseffekten zurückgehalten.

Ahnliches gilt für die Vorrichtungen zur Abtrennung von Blutkomponenten der EP 0 312 595. Die EP 0 312 595 mit Baumwollfäden gefüllte zylindrische Gehäuse mit einer Einlasseinrichtung und einer Auslasseinrichtung für Blut. Bei den Baumwollfäden handelt es sich um Stapelfasern, die entsprechend den Figuren der EP 0 312 595 in zwei Lagen aus Aggregaten von ungeordneten Wirrfasern angeordnet sind. Die Blutkomponenten wie z.B. weiße Blutkörperchen oder Blutplättchen, werden über Größenausschluss in den Zwischenräumen zwischen den Fasern der Faseraggregate zurückgehalten.

Gemäß EP 1 230 940 wird ebenfalls ein Filter für die Entfernung von Leukozyten aus Blut verwendet. Der Filter kann ein Vlies, ein Gewebe oder eine poröse Flach- bzw. Hohfasermembran sein. Das zu behandelnde Blut strömt durch das Vlies, Gewebe oder die poröse Membran. Das Filtermaterial weist ein Coating aus einem hydrophilen synthetischen Polymer auf. Dieses Coating erlaubt den Durchgang von Thrombozyten durch den Filter bei gleichzeitiger Entfernung von Leukozyten durch Adsorption.

WO 95/18665 offenbart einen Filter und ein Verfahren zur Entfernung von Leukozyten und virusinaktivierenden Stoffen aus Plasma oder anderen Blutfraktionen. Der Filter basiert auf einem Netz aus textilen Fasern. An dem Netz sind Liganden mit hoher Affinität zu virusinaktivierenden Stoffen bzw. Leukozyten kovalent gebunden. Es handelt sich hierbei um eine selektive, aber technisch sehr aufwändige Methode, da die Liganden direkt oder über Linker an eine Polymermatrix zu binden sind.

In der US 4,925,572 wird ein Filter beschrieben, der zur Entfernung von Leukozyten von einem Blutprodukt durchströmt wird und bei dem entsprechend der Zusammenfassung mittels eines porösen Elements Leukozyten sowohl über Adsorption als auch über Filtration entfernt werden. Bei den porösen Elementen kann es sich vorzugsweise um aus Fasern aufgebaute Elemente handeln, wobei diese porösen Elemente einen Porendurchmesser im Bereich von ca. 4 bis ca. 8 µm aufweisen können. Das poröse Element zu Entfernung von Leukozyten kann oberflächenmodifiziert sein, um so eine bestimmte Oberflächenspannung und damit eine gute Benetzbarkeit des Elements zu generieren. Die US 4,925,572 strebt eine Entfernung der Leukozyten hautsächlich über Adsorption an.

Die Rückhaltung der Leukozyten mit solchen Filtern beruht auf einem Zelltrapping im Faservlies sowie einer mehr oder weniger starken Adsorption der Zellen an der Faseroberfläche. Allerdings werden bei diesen Verfahren die verschiedenen Blutzellen mechanisch sehr stark belastet, was zu einer Zellaktivierung oder gar zur Zerstörung der Blutzellen führen kann.

Ein wesentlicher Nachteil der bestehenden Systeme und Verfahren besteht darin, dass die verschiedenen Zelltypen nicht gezielt oder spezifisch adsorbiert werden können und dass neben den Monocyten und Granulozyten auch Lymphozyten, Thrombozyten und Erythrocyten adsorbiert werden. Dies kann, je nach Indikation, entweder gar nicht notwendig oder sogar schädlich für den Patienten sein. Einen Spezialfall stellt die Adsorption von Thrombozyten dar. Thrombozyten sind nach Aktivierung zentral an der Blutgerinnung beteiligt. Damit es während des extrakorporalen Kreislaufes nicht zur Blutgerinnung kommt, muß, etwa durch Gabe von Heparin als Antikoagulanz, medikamentös gegengesteuert werden. Kommt es trotz Antikoagulanz zur Blutgerinnung, führt das zu einem verstopften Filter.

Ein weiterer Nachteil von konventionellen Leukocytenfiltern besteht in der häufig schwierigen Handhabung vor dem klinischen Einsatz, z.B. in Bezug auf Entlüftung. Luftblasen stellen in einem extrakorporalen Kreislauf potentiell eine Gefährdung des Patienten dar, sind also sehr unerwünscht. Je einfacher eine Entfernung des im Filters vorhandenen Luft möglich ist, desto besser die Handhabung und sicherer die Anwendung.

Aufgabe der vorliegenden Erfindung ist es daher, ein Verfahren zur Reduzierung von Leukozyten aus Blut zur Verfügung zu stellen, bei dem die Nachteile des Standes der Technik zumindest verringert sind, bei dem das Blut schonend behandelt wird und das Alternativen zulässt, um verschiedene Zelltypen gezielt zu entfernen. Es ist des Weiteren Aufgabe der vorliegenden Erfindung, eine Vorrichtung zur Durchführung eines solchen Verfahrens zur Verfügung zu stellen.

Diese Aufgabe wird gelöst durch ein Verfahren zur Reduzierung der Leukozytenzahl in Blut durch Behandlung des Blutes in einer Anordnung einer Vielzahl von Fäden auf Basis organischer Polymere, wobei die Vielzahl von Fäden in einem Gehäuse mit einer Einlasseinrichtung und einer Auslasseinrichtung gehalten werden, das Blut über die Einlasseinrichtung dem Gehäuse zugeführt wird, anschließend das Gehäuse durchströmt, wobei das Blut die Fäden an ihrer äußeren Oberfläche umströmt, und schließlich über die Auslasseinrichtung das Gehäuse wieder verlässt, dadurch gekennzeichnet, dass Fäden eingesetzt werden, die bei Umströmung ihrer äußeren Oberfläche mit Blut eine Bildung des Komplementaktivierungsproduktes C5a in einer Konzentration von mindestens 10 µg pro m² Fadenoberfläche bewirken und dass die Anordnung der Fäden einen hohen Grad an Ordnung aufweist, wobei unter einem hohen Grad an Ordnung zu verstehen ist, dass ein hoher Anteil der Fäden entlang ihrer Erstreckungsrichtung nebeneinander angeordnet sind.

Die Erfindung umfasst ebenfalls eine Vorrichtung zur Reduzierung der Leukozytenzahl in Blut umfassend eine Vielzahl von Fäden auf Basis organischer Polymere in einem Gehäuse mit einer Einlasseinrichtung und einer Auslasseinrichtung, wobei die Fäden mit zumindest einem ihrer Enden so in eine mit der Gehäuseinnenseite verbundenen Vergussmasse eingebettet sind, dass um die Fäden herum ein von Blut durchströmbarer Außenraum ausgebildet ist, dadurch gekennzeichnet, dass die Anordnung der Fäden einen hohen Grad an Ordnung aufweist, wobei unter einem hohen Grad an Ordnung zu verstehen ist, dass ein hoher Anteil der Fäden entlang ihrer Erstreckungsrichtung nebeneinander angeordnet sind, und dass die Fäden auf Basis organischer Polymere eine Bildung des Komplementaktivierungsproduktes C5a in einer Konzentration von mindestens 10 µg pro m² Fadenoberfläche bewirken.

C5a ist ein Spaltprodukt des Plasmaproteins C5. Der Maximalwert der C5a Konzentration im Blut ist daher limitiert durch die Konzentration von C5 im

Blutplasma, wobei die C5 Konzentration im Plasma starken individuellen Schwankungen unterliegt und etwa 40mg/l bis 150 mg/l betragen kann. Aufgrund des Molmassenverhältnisses von C5 zu C5a ergibt sich dadurch eine theoretische Maximalkonzentration von C5a im Blut von 9 mg/l.

Die Konzentration des Komplementaktivierungsproduktes C5a wird in Blutplasma ermittelt unter Verwendung eines Sandwich-ELISA (enzymgekoppelter Immunnachweis) der Firma DRG Diagnostics, Marburg. Nach Kontakt der Fäden mit humanem Spenderblut (5U/ml Heparin) wird zu verschiedenen Zeiten 1.8 ml Blut entnommen und mit 0.2 ml 100 mM EDTA-Lösung abgestoppt. Vor der Analyse wird entsprechend der Herstellervorschrift C5 ausgefällt (200µl Plasma + 200 µl Präzipitationsreagens). 50 µl Überstand wird in die Bestimmung eingesetzt. Die Nachweisempfindlichkeit des Assays liegt bei < 0.02 µg/l, die Wiederfindungsrate von C5a im Plasma bei 86-114% und der Variationskoeffizient bei 5-8% (Intraassay) bzw. 6-10% (Interassay). Die gemessene C5a Konzentration ist vom Blutvolumen und der Fadenoberfläche abhängig. Daher ist zur Ermittlung der C5a Konzentration bezogen auf die äußere Oberfläche der Fäden der absolute C5a Gehalt in der Probe zu bestimmen und in Relation zur äußeren Fadenoberfläche zu setzen. Dabei ist ein Verhältnis Blutvolumen (V) zu Fadenoberfläche (A) V/A von 0,3 L/m² einzuhalten. Die Ermittlung der flächenbezogenen C5a Konzentration erfolgt nach einer Behandlungsdauer von 3 h, d.h. die Blutprobe wird 3 h an der äußeren Oberfläche der Fäden entlang geführt, wobei eine lineare Strömungsgeschwindigkeit von 5 bis 30 cm/min einzuhalten ist. Da die Messergebisse spenderabhängig individuellen Schwankungen unterliegen, sollte die Stichprobenanzahl N mindestens 2 betragen und die Mittelwerte der Stichproben angegeben werden.

Ohne an die Theorie gebunden sein zu wollen, wird vermutet, dass bei der Behandlung entzündlicher Erkrankungen der Komplementaktivierung eine wichtige Bedeutung zukommt und dass eine Reduzierung der Leukozytenzahl in Kombination mit einer Komplementaktivierung wesentlich effektiver ist als eine Reduzierung der Leukozytenzahl allein. Dazu muss die Komplementaktivierung, bestimmt durch die Konzentration von C5a im Blut, oberhalb des erfindungsgemäßen Schwellenwertes liegen.

Ein Zusammenhang zwischen den Parametern Leukozytenzahl und C5a ist wahrscheinlich dadurch gegeben, dass bestimmte Leukozyten durch C5a aktiviert werden können. Die Aktivierung durch C5a und andere Faktoren bewirkt, dass die Zellen adhäsiver ("klebriger") werden und daher verstärkt an C5a erzeugende Oberflächen binden.

Es ist daher bevorzugt, dass die Fäden eine weiter verstärkte Bildung des Komplementaktivierungsproduktes C5a in einer Konzentration von mindestens 75 µg pro m² Fadenoberfläche bewirken.

Besonders bevorzugt bewirken die Fäden eine Bildung des Komplementaktivierungsproduktes C5a in einer Konzentration von mindestens 100 µg pro m² Fadenoberfläche.

Um eine Schädigung der im Blut enthaltenen Zellen zu vermeiden ist es wichtig, dass das Blut im wesentlichen nicht in das Fadenmaterial eindringt bzw. nicht durch das Fadenmaterial hindurchströmt. Bevorzugt ist daher ein Fadenmaterial bzw. sind daher Fäden mit einer dichten Oberfläche oder im Falle einer porösen Oberfläche mit einer maximalen Porenweite von 0,1 µm.

Die Fäden auf Basis organischer Polymere können Multifilament-Fäden sein, d.h. Fäden, die aus einer Vielzahl von Einzelfilamenten bestehen, bevorzugterweise sind die Fäden Monofilament-Fäden, d.h. sie bestehen aus einem einzigen Filament.

Da die für das erfindungsgemäße Verfahren erforderliche C5a Bildung nicht nur abhängig ist vom Polymer, sondern auch von Beimischungen oder vom Substitutionsgrad des Polymers, umfasst die Bezeichnung "auf Basis organischer Polymere" die polymeren Werkstoffe als solche, Substitutionen, Mischungen davon, Copolymere dieser Werkstoffe sowie evtl. zugegebene Hilfsstoffe oder Additive, wie z.B. Hydrophilierungsmittel.

Unter einem hohen Grad an Ordnung im Sinne der vorliegenden Erfindung ist zu verstehen, dass die Fäden in ähnlicher Anordnung zueinander liegen bzw. dass ein hoher Anteil der Fäden entlang ihrer Erstreckungsrichtung nebeneinander angeordnet sind. Theoretisch hat ein Bündel gerader Fäden, die parallel zueinander liegen, den höchsten Grad an Ordnung. Einen hohen Grad an Ordnung im Sinne der vorliegenden Erfindung weist auch ein Bündel gewellter bzw. ondulierter Fäden auf, wobei die Fäden des Fadenbündels alle die gleiche Erstreckungsrichtung aufweisen. Auch ist unter einem hohen Grad an Ordnung im Sinne der vorliegenden Erfindung ein Faserbündel umfasst, das in einer Schlaufe gelegt ist. Auch hier ist die Anordnung der Fäden zueinander ähnlich. Ebenfalls ist unter einem hohen Grad an Ordnung zu verstehen dass mindestens 30% der Fäden parallel liegen. Des weiteren sind Fäden umfasst, die in mehreren Lagen vorliegen, wobei die Fäden innerhalb einer Lage im wesentlichen parallel zueinander angeordnet sind. Die parallelen Fäden einer Lage können sich allerdings mit den parallelen Fäden einer anderen Lage kreuzen. Derartige Anordnungen sind in der EP 285 812 beschrieben. Von den erfindungsgemäßen Anordnungen mit einem hohen Grad an Ordnung nicht umfasst sind Vliese oder Wirrfasermatten, in denen die Fasern völlig ungeordnet vorliegen und miteinander vermischt sind. Im Vergleich zu Vliesen weist die erfindungsgemäße Anordnung der Fäden mit einem hohen Grad an Ordnung eine höhere Oberfläche und bei Anwendung des erfindungsgemäßen Verfahrens eine gleichmäßige Blutfilmdicke auf. Der hohe Grad an Ordnung sorgt dafür, dass das an den Fäden vorbeiströmende Blut vergleichsweise geringe Turbulenzen aufweist und die im Blut enthaltenen Zellen einer geringen Scherbelastung ausgesetzt sind. Zudem sorgt der hohe Grad an Ordnung dafür, dass die Bildung von Toträumen und bevorzugte Kanälen, sog. Shunts, weitgehend vermieden wird. Hierdurch wird eine besonders schonende Blutbehandlung erreicht.

Der hohe Grad an Ordnung sorgt auch dafür, dass die Reduzierung der Leukozytenzahl im Wesentlichen nicht durch einen Siebeffekt hervorgerufen wird, wie dies z.B. bei einem Vlies der Fall wäre, sondern durch Adsorptionseffekte, wodurch eine besonders schonende Blutbehandlung ermöglicht wird. Zudem bewirkt der bei einem Vlies auftretende Siebeffekt auch zwangsläufig eine ungewollte Reduzierung anderer zellulärer Blutsbestandteile, z. B. der Thrombozyten.

Für das erfindungsgemäße Verfahren bzw. die erfindungsgemäße Vorrichtung eignen sich nicht nur Vollfäden, es können auch Hohlfäden eingesetzt werden. Hohlfäden weisen eine innere und eine äußere Oberfläche auf. Bei der Verwendung von Hohlfäden sind diese, wie die Vollfäden auch, entlang der äußeren Oberfläche anzuströmen. Die Hohlfäden können vor oder nach der Umströmung der äußeren Oberfläche auch in ihrem Lumen vom Blut durchströmt werden. Es hat sich jedoch gezeigt, dass bei zusätzlicher Durchströmung des Lumens keine Vorteile erzielt werden. Natürlich können als Hohlfäden auch Hohifasermembranen mit dichter oder poröser Struktur eingesetzt werden. Es ist dabei darauf zu achten, dass das erfindungsgemäße Verfahren so durchgeführt wird, dass das Blut im wesentlichen nicht in den Hohtfaden bzw in die Hohlfasermembran eindringt und die Membranwand durchströmt.

Die Anzahl der Fäden liegt bevorzugt im Bereich von 2000 bis 20000 Fäden, besonders bevorzugt im Bereich von 4000 bis 14000 Fäden. Der Außendurchmesser der Fäden sollte zwischen 0,05 mm und 2 mm liegen, vorzugsweise zwischen 0,1 mm und 2 mm, besonders bevorzugt zwischen 0,2 mm und 1 mm.

Bevorzugt weist die Anordnung der Fäden eine spezifische Oberfläche zur Blutbehandlung zwischen 0,1 und 100 cm² Fadenoberfläche pro ml zu behandelnden Blutes, vorzugsweise zwischen 0,5 und 20 cm² Fadenoberfläche pro ml zu behandelnden Blutes auf. Die Menge des zu behandelnden Blutes ergibt sich aus der Dauer der Blutbehandlung und dem Volumenstrom.

Als Fäden aus organischen Polymeren kommen Fäden aus natürlichen Polymeren oder aus Polymeren, die auf synthetischen Wege hergestellt wurden in Frage. Fäden aus natürlichen Polymeren sind insbesondere solche auf Basis von zellulosischen Polymeren, was Fäden, die sog. polymeranalogen Reaktionen unterzogen worden sind, ebenfalls umfasst. Beispiele für solche Fäden auf Basis von Zellulose sind solche aus regenerierter Zellulose, Zelluloseazetat oder modifizierter Zellulose wie z.B. Zelluloseester, Zelluloseäther, mit Benzylgruppen modifizierte Zellulose (Benzylzellulose) oder mit Diethylaminoethyl modifizierte Zellulose oder Mischungen dieser zellulosischen Polymere. Mit Fäden auf Basis von zellulosischen Polymeren wird im erfindungsgemäßen Verfahren eine hohe Reduzierung der Leukozytenzahl erreicht, eine besonders hohe Reduzierung wird mit Fäden aus regenerierter Zellulose erhalten. Des Weiteren können auch Fäden auf der Basis von Chitin, bzw. Chitosan zum Einsatz kommen.

Bei den organischen Polymeren handelt es sich auch um solche Polymere, die auf synthetischem Wege hergestellt werden. Für Fäden aus synthetischen Polymeren können solche verwendet werden, die aus Polyolefinen, Polyamiden, Polyacrylnitril, Polycarbonaten oder Polyester sowie daraus gewonnen Modifikationen, Blends, Mischungen oder Copolymere dieser Polymere bestehen. Vorzugsweise werden solche verwendet, die auf Sulfonpolymeren, wie z.B. Polysulfon oder Polyethersulfon, basieren. Diesen Polymeren können weitere Polymere wie z.B. Polyethylenoxid, Polyhydroxyether, Polyethylenglykol, Polyvinylalkohol oder Polycaprolacton als Zusatzstoffe beigemischt werden. Die Fäden können darüber hinaus noch eine Beschichtung mit einem Additiv aufweisen. Bevorzugt enthalten solche Fäden ein Hydrophilierungsmittel, z.B. Polyvinylpyrrolidon oder auch hydrophile Modifikationen dieser Polymere.

Selbstverständlich eignet sich das Verfahren nicht nur zur Reduzierung der Leukozytenzahl in Vollblut, sondern auch zur Reduzierung der Restleukozytenzahl in Blutplasma oder anderen Blutkonzentraten. Daher wird im Rahmen der vorliegenden Erfindung unter Blut Vollblut, Blutplasma oder ein Blutkonzentrat verstanden.

Es hat sich gezeigt, dass mit den angegebenen Fadenmaterialien in erster Linie Leukozyten reduziert werden. Insbesondere mit zellulosischen Fadenmaterialien wird hauptsächlich die Anzahl von Granulozyten und Monozyten reduziert. Lymphozyten werden mit zellulosischen Materialien nur geringfügig reduziert.

Es ist daher im Rahmen des erfindungsgemäßen Verfahrens möglich, gezielt bestimmte Zelltypen einer Klasse zu reduzieren, wie aus der Klasse der Leukozyten die Monocyten und Granulozyten und nicht die Lymphocyten. Weiterhin zeichnen sich Fadenmaterialien auf Basis von Zellulose dadurch aus, dass Thrombozyten nur in geringem Maße zurückgehalten werden.

Um sicher zu gewährleisten, dass alle Fäden gleichermaßen mit dem an den Fäden entlangströmenden Blut in Kontakt kommen, können die Fäden zueinander beabstandet angeordnet werden, beispielsweise über sog. Abstandsfäden (Spaceryarn). Eine Beabstandung der Fäden ist im Hinblick auf die Vermeidung eines Siebeffektes besonders vorteilhaft. Solche Abstandsfäden sind besonders vorteilhaft, da dadurch ein gleichmäßiger Abstand zwischen den im wesentlichen parallel vorliegenden Fäden gewährleistet wird. Anordnungen mit solchen Abstandsfäden werden beispielsweise in der EP 732 141 oder der EP 285 812 beschrieben. Vorzugsweise bestehen die Abstandsfäden aus dem gleichen Material wie die Vielzahl paralleler Fäden. Es ist aber auch möglich, durch Verwendung eines anderen Fadenmaterials als Abstandsfaden, die Anzahl weiterer im Blut enthaltener Zelltypen zu reduzieren.

Für bestimmte Anwendungsfälle könnte es vorteilhaft sein, zusätzlich Thrombozyten gezielt aus dem Blut zu entfernen. Für diese Anwendungsfälle eignen sich Fäden aus Polyethylenterephtalat (PET), Polysulfon oder Polyethersulfon. Sollte eine Reduzierung von Thrombozyten und Leukozyten gewünscht sein, bietet sich eine Kombination beispielsweise von Zellulosefäden und PET-Fäden für das erfindungsgemäße Verfahren an.

Wie bereits beschrieben befindet sich die Vielzahl von Fäden auf Basis organischer Polymere bevorzugt in einem Gehäuse mit einer Einlasseinrichtung und einer Auslasseinrichtung. Hierzu sind die Fasern in der Regel so auf bekannte Weise mit zumindest einem ihrer beiden Enden in eine mit der Gehäuseinnenseite verbundenen Vergussmasse eingebettet, dass um die Fasern herum ein vom Blut durchströmbarer Außenraum ausgebildet wird. Beispielsweise können die Fasern mit ihren Enden getrennt in Vergussmassen eingebettet sein und sich zwischen diesen Vergussmassen im wesentlichen geradlinig erstrecken. Ein solcher Aufbau ist beispielsweise bei üblichen im sog. "cross-flow" Modus betriebenen Hohlfasermodulen realisiert und unter anderem bei handelsüblichen Dialysemoduln zu finden. Bei derartigen Moduln wird bei Durchführung des erfindungsgemäßen Verfahrens der Außenraum um die Hohlfasern vom Blut durchströmt. Die Fäden können aber auch nur mit einem Ende eingebettet sein oder mit ihren beiden Enden in derselben Vergussmasse eingebettet sein, und das andere Ende bzw. die sich ausbildende Schlaufe frei ohne Einbettung umströmt werden. Derartige Fadenanordnungen werden beispielsweise in der EP 732 142 oder der EP 371 189 beschrieben.

Der Füllgrad des Gehäuses mit den erfindungsgemäßen Fäden aus organischen Polymeren sollte zwischen 10% und 70%, bevorzugt zwischen 30% und 60% betragen. Da die Fäden, abhängig vom Fadenmaterial, beim Kontakt mit Flüssigkeit unterschiedlich stark quellen können, ist die Bestimmung des Füllgrades des Gehäuses mit Fäden in gequollenem Zustand zu ermitteln. Bei stark quellenden Fäden, wie beispielsweise solche auf Zellulosebasis, sind deutliche Unterschiede bezüglich des Fadendurchmessers in gequollenem bzw. nicht gequollenem Zustand zu beobachten. Aufgrund der Quellung ergeben sich daher unterschiedliche Füllgrade, wenn die Fäden in trockenem Zustand vorliegen. Fäden, die nicht oder nahezu nicht quellen, wie beispielsweise solche aus Polysulfon zeigen dagegen keinen oder nur einen geringen Unterschied bei der Bestimmung des Füllgrades in gequollenem bzw. nicht gequollenem Zustand.

Der Füllgrad des Gehäuses ist in dem vorgegebenen Bereich zu limitieren, einerseits um eine hinreichend große Faseroberfläche zur Verfügung zu stellen, andererseits um einen Siebeffekt bei der Reduzierung von Leukozyten in dem erfindungsgemäßen Verfahren zu vermeiden.

Die Verweilzeit des Blutes im Gehäuse sollte mindestens 0,5 min betragen und eine Dauer von 5 min nicht überschreiten. Bei langen Verweilzeiten über 5 min nimmt die Gerinnungsneigung des Blutes zu und trotz Zugabe eines Antikoagulanz erhöht sich die Gefahr der Blutgerinnung. Bei geringen Verweilzeiten unterhalb 0,5 min ist hingegen die Leukozytenadsorption nicht ausreichend. Bevorzugt ist daher eine Verweilzeit des Blutes im Gehäuse von 1 min bis 3 min.

Die lineare Fließgeschwindigkeit des Blutes durch das Gehäuse mit der Vielzahl von Fäden auf Basis organischer Polymere soll zwischen 5 und 30 cm/min liegen. Unter der linearen Fließgeschwindigkeit ist die mittlere Geschwindigkeit zu verstehen, mit der das Blut von der Einlasseinrichtung zur Auslasseinrichtung durch das Gehäuse strömt. In die Berechnung der linearen Fließgeschwindigkeit geht der sog. freie Strömungsquerschnitt ein, der sich aus der Querschnittsfläche im Inneren des Gehäuses abzüglich der Summe der Querschnittsflächen aller Fäden im Gehäuse zusammensetzt. Hierbei ist zu beachten, dass zur Berechnung der Querschnittsfläche eines Fadens der Außendurchmesser des Fadens herangezogen werden muss, wobei bei in Blut quellbaren Fadenmaterialien der Außendurchmesser in gequollenem Zustand maßgeblich ist. Lineare Fließgeschwindigkeiten niedriger als 5 cm/min begünstigen eine Thrombozytenaggregation und Blutgerinnung. Fließgeschwindigkeiten oberhalb 30 cm/min behindern die Adsorption der zellulären Blutbestandteile an die Fadenoberfläche.

Wie bereits ausgeführt, weisen die Fäden auf Basis organischer Polymere in ihrer Anordnung einen hohen Grad an Ordnung auf. In einer bevorzugten Ausführungsform des erfindungsgemäßen Verfahrens bzw. der erfindungsgemäßen Vorrichtung liegen die Fäden auf Basis organischer Polymere als Fadenbündel von im wesentlichen parallelen Fäden vor. In einer weiteren bevorzugten Ausführungsform des erfindungsgemäßen Verfahrens liegen die Fäden auf Basis organischer Polymere in einer oder mehrerer Lagen vor, wobei die Fäden innerhalb einer Lage im wesentlichen parallel liegen

Die Erfindung wird anhand der nachfolgenden Beispiele und Figuren näher erläutert.

Es zeigen:
- Figur 1:: Reduktion der Leukozytenzahl in einer Blutprobe durch das erfindungsgemäße Verfahren unter Verwendung von Hohlfäden aus regenerierter Zellulose in Abhängigkeit von der Zeit
- Figur 2:: Reduktion der Granulozytenzahl, Lymphozytenzahl, Monozytenzahl und Thrombozytenzahl in einer Blutprobe behandelt mit dem erfindungsgemäßen Verfahren unter Verwendung von Hohlfäden aus regenerierter Zellulose in Abhängigkeit von der Zeit
- Figur 3:: Konzentration des Komplementaktivierungsproduktes C5a in einer Blutprobe behandelt mit dem erfindungsgemäßen Verfahren unter Verwendung von Hohlfäden aus regenerierter Zellulose in Abhängigkeit von der Zeit
- Figur 4:: Reduktion der Granulozytenzahl, Lymphozytenzahl, Monozytenzahl und Thrombozytenzahl in einer Blutprobe behandelt mit dem erfindungsgemäßen Verfahren unter Verwendung von Hohlfäden aus regenerierter Zellulose und zusätzlicher Verwendung von PET-Fäden in Abhängigkeit von der Zeit
- Figur 5:: Reduktion der Leukozytenzahl in einer Blutprobe behandelt mit dem erfindungsgemäßen Verfahren unter Verwendung von Hohlfäden aus modifizierter Zellulose in Abhängigkeit von der Zeit
- Figur 6:: Konzentration des Komplementaktivierungsproduktes C5a in einer Blutprobe behandelt mit dem erfindungsgemäßen Verfahren unter Verwendung von Hohlfäden aus modifizierter Zellulose in Abhängigkeit von der Zeit
- Figur 7:: Reduktion der Leukozytenzahl in einer Blutprobe behandelt mit dem erfindungsgemäßen Verfahren unter Verwendung von Hohlfäden aus Polyethersulfon in Abhängigkeit von der Zeit
- Figur 8:: Reduktion der Leukozytenzahl in einer Blutprobe behandelt mit dem erfindungsgemäßen Verfahren unter Verwendung von Hohlfäden aus Polyethersulfon und zusätzlicher Verwendung von PET-Fäden in Abhängigkeit von der Zeit
- Figur 9:: Konzentration des Komplementaktivierungsproduktes C5a in einer Blutprobe behandelt mit dem erfindungsgemäßen Verfahren unter Verwendung von Hohlfäden aus Polyethersulfon und zusätzlicher Verwendung von PET-Fäden in Abhängigkeit von der Zeit

### Beispiel 1

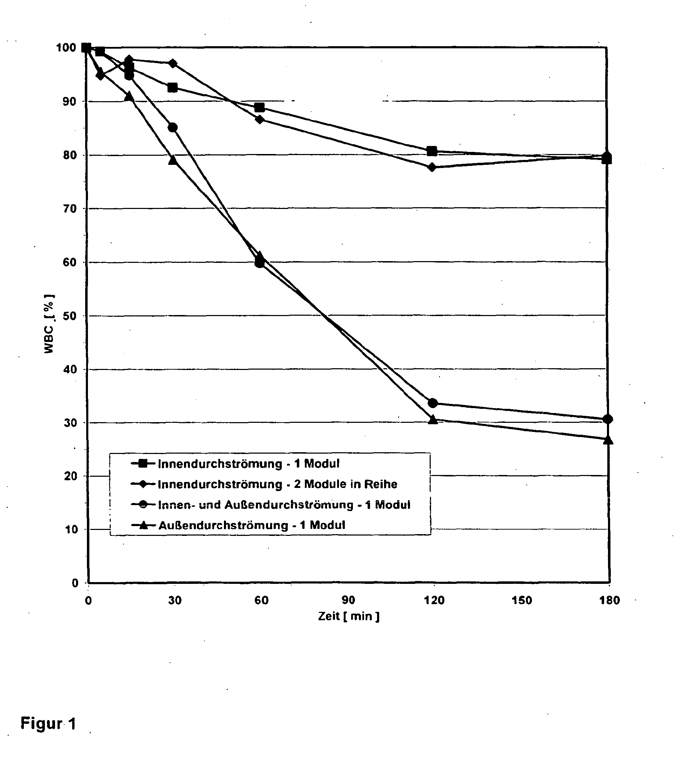

Unter Verwendung von Hohlfasern aus regenerierter Zellulose wurden Blutproben mit dem erfindungsgemäßen Verfahren behandelt und die Leukozytenzahl, die Anzahl bestimmter Leukozytentypen, die Thrombozytenzahl sowie die Bildung des Komplementaktivierungsproduktes C5a in Abhängigkeit von der Zeit bestimmt. Figur 1 zeigt die relative prozentuale Reduktion der Leukozytenzahl (WBC-White Blood Cells) in einer Blutprobe behandelt mit dem erfindungsgemäßen Verfahren. Verwendet wurde dafür eine Schar Hohlfäden aus regenerierter Zellulose mit einer äußeren Oberfläche von 1,36 m² und einer inneren Oberfläche von 1,27m². Die Hohlfadenschar bestehend aus 7400 Fäden mit einem Fadendurchmesser von 242µm in gequollenem Zustand und einer Länge von 260mm wurde dafür in einem zylindrischen Gehäuse mit einem Innendurchmesser von 33mm angeordnet. Der Füllgrad des Gehäuses betrug ca. 40%. Als Blutprobe wurde 410 ml Humanblut verwendet, das über eine Dauer von maximal 3h mit einem Volumenstrom von 50ml/min zirkuliert wurde, was einer linearen Fließgeschwindigkeit von 10 cm/min entspricht. Die Verweilzeit des Blutes im Gehäuse betrug 2,5min. Die in Figur 1 dargestellten Untersuchungsergebnisse zeigen deutlich, dass bei Strömung des Blutes entlang der äußeren Oberfläche der Fäden eine ausgeprägte Reduktion der Leukozytenzahl stattfindet. Eine Innendurchströmung führt nur zur einer geringen Reduzierung der Zellzahl von ca. 15% der Ausgangsmenge. Innen- und Außenanströmung bringen keinen Vorteil gegenüber einer alleinigen Außenanströmung. Auch eine Innendurchströmung von 2 Moduln hintereinander führt nicht zu einer Leukozytenreduktion in dem Maße wie sie mit Außendurchströmung erreicht werden kann, obwohl die Kontaktfläche des Blutes mit dem Fadenmaterial mit 2,54 m² deutlich größer ist als bei Außenanströmung (1,36 m²). Aus Figur 1 ist ersichtlich, dass eine Reduktion der Zellzahl nur erhalten wird, wenn das Blut außen an den Hohlfäden vorbeiströmt. Innendurchströmte Hohlfäden zeigen keine oder nur eine sehr geringe Zelladhäsion. Dies gilt auch, wenn Innendurchströmumg mit einer Außenumströmung kombiniert wird.

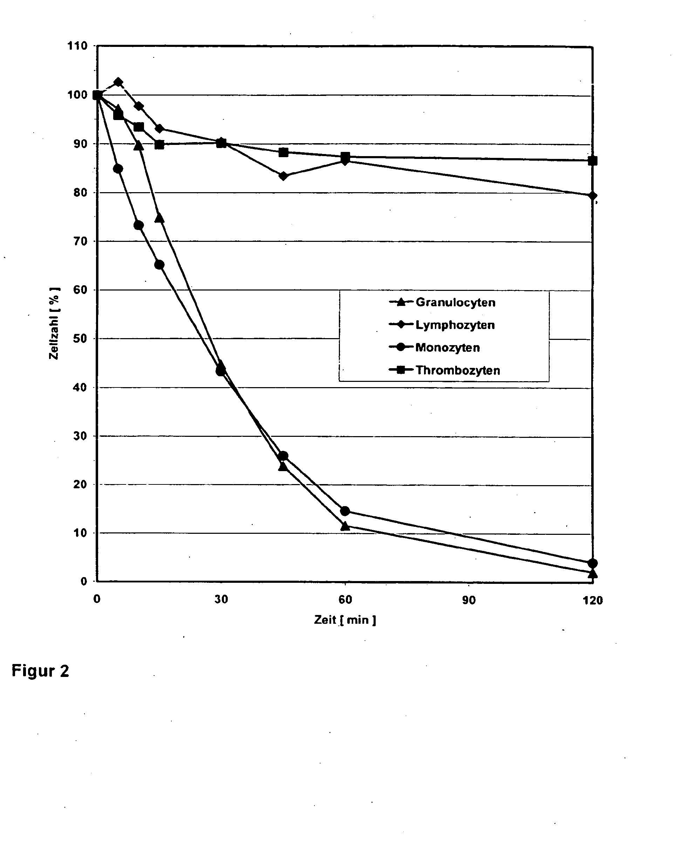

Figur 2 zeigt, in welchem Maß die Anzahl der einzelnen Leukozytentypen reduziert werden und welche Auswirkung das erfindungsgemäße Verfahren auf die Anzahl der Thrombozyten hat. In Figur 2 ist die relative prozentuale Reduktion der Zellzahl von Monocyten, Granulocyten, Lymphocyten und Thrombozyten in Abhängigkeit von der Blutbehandlungszeit dargestellt, die unter Verwendung von Hohlfäden aus regenerierter Zellulose mit einer äußeren Oberfläche von 0,56 m² und 240 ml Humanblut ermittelt wurden. 5400 Fäden mit einem Fadendurchmesser von 275µm in gequollenem Zustand und einer Länge von 120mm wurden dafür in einem zylindrischen Gehäuse mit einem Innendurchmesser von 38mm angeordnet. Der Füllgrad des Gehäuses betrug ca. 29%. Die Blutprobe wurde über eine Dauer von maximal 3h mit einem Volumenstrom von 117 ml/min zirkuliert, was einer linearen Fließgeschwindigkeit von 14 cm/min entspricht. Die Verweilzeit des Blutes im Gehäuse betrug ca. 0,8 min. Monocyten und Granulocyten werden zu über 95% nach 3h Dauer aus der Blutprobe entfernt, weniger jedoch Lymphocyten und Thrombozyten, die nur zu ca. 20% bzw. 15% reduziert werden. Durch Verwendung von regenerierter Zellulose lassen sich also speziell Monozyten und Granulozyten aus Blut entfernen.

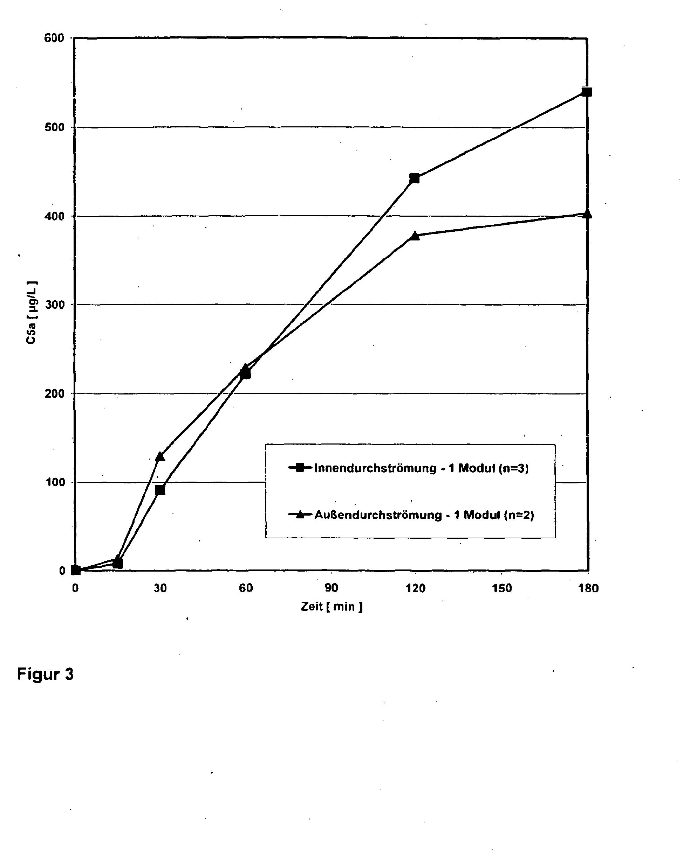

Figur 3 zeigt die Konzentration des Komplementaktivierungsproduktes C5a in einer mit dem erfindungsgemäßen Verfahren behandelten Blutprobe. Die Untersuchungen sind unter den gleichen Bedingungen, wie unter Figur 1 dargestellt, ermittelt worden. Nach einer Blutbehandlung von 3 Stunden wurde bei Außenumströmung ein C5a Gehalt in der behandelten Probe von 403µg/l ermittelt. Das entspricht einer C5a Bildung von 165 µg in der behandelten Blutprobe, bzw. von 121 µg C5a pro m² Fadenoberfläche.

### Beispiel 2

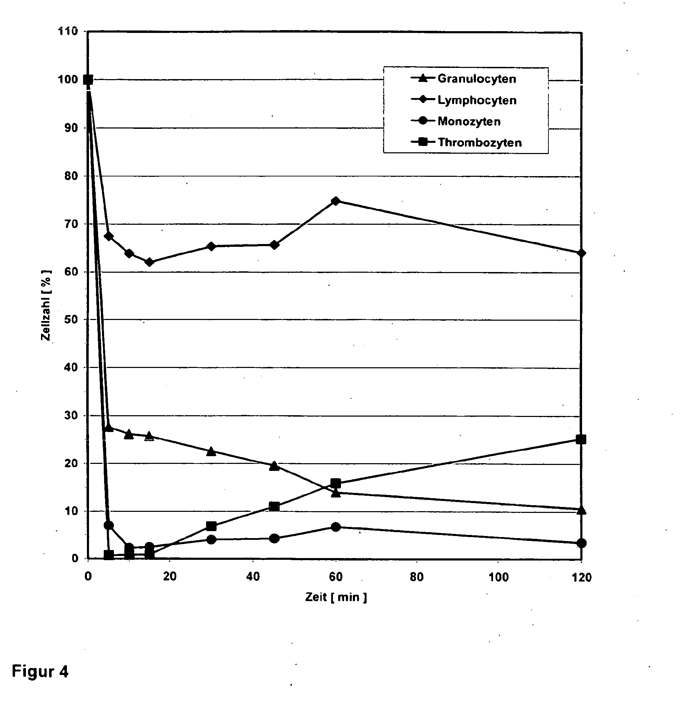

Es wurde ein Hohlfasermodul wie unter Beispiel 1, Figur 2 beschrieben, eingesetzt, wobei jedoch das Hohlfaserbündel zusätzlich Polyethylenterephthalat (PET) Fäden enthielt. Figur 4 zeigt die zeitabhängige relative prozentuale Reduktion der Zelllzahl von Monocyten, Granulocyten, Lymphocyten und Thrombozyten bei zusätzlicher Verwendung von Polyethylenterephthalat-Fäden. Die Versuchsbedingungen entsprechen ansonsten den in Beispiel 1 bezüglich Figur 2 dargestellten Versuchsbedingungen. Durch die zusätzliche Verwendung von Polyethylenterephthalat Fäden wird eine viel schnellere Entfernung von Monocyten und Granulocyten im Vergleich zur ausschließlichen Verwendung von Fäden aus regenerierter Zellulose erreicht. Bereits nach 15 Minuten sind über 95% der Monozyten und 75% der Granulozyten aus der Probe entfernt. Lymphozyten werden zu ca. 60% aus der Probe entfernt und Thrombozyten zu etwa 75%.

### Beispiel 3

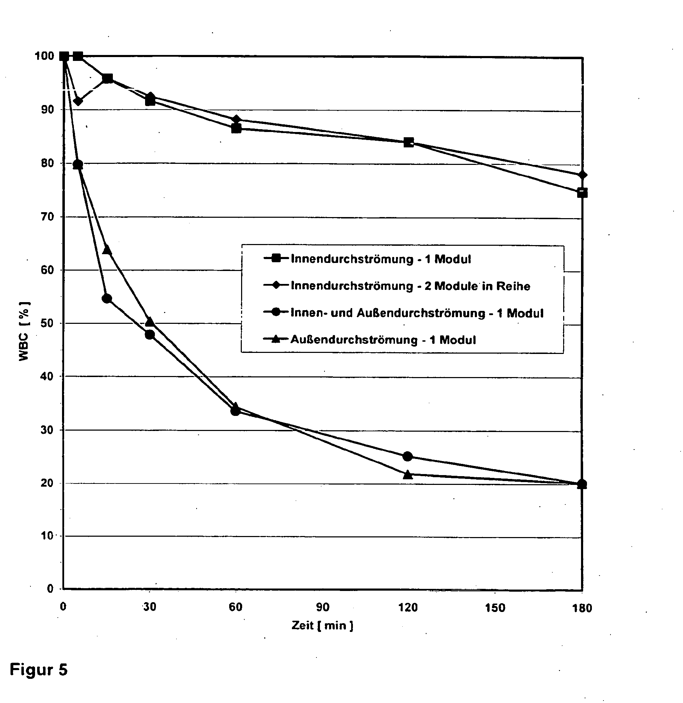

Unter Verwendung von kommerziell erhältlichen Hohlfasermembranen aus mit Benzylgruppen modifizierter Zellulose (Benzylzellulose) wurden Blutproben mit dem erfindungsgemäßen Verfahren behandelt und die Leukozytenzahl sowie die Bildung des Komplementaktivierungsproduktes C5a in Abhängigkeit von der Zeit bestimmt. Figur 5 zeigt die zeitabhängige relative prozentuale Reduktion der Leukozytenzahl (WBC - White Blood Cells) im Verhältnis zur Ausgangsmenge in einer Blutprobe behandelt mit dem erfindungsgemäßen Verfahren. Verwendet wurden hier Hohlfäden mit einer inneren Oberfläche von 1,36 m² und einer äußeren Oberfläche von 1,47 m². Die behandelte Blutprobe war 440 ml Humanblut. Eine Fadenschar von 7900 Fäden mit einem Fadendurchmesser von 246µm in gequollenem Zustand und einer Länge von 240mm wurde dafür in einem zylindrischen Gehäuse mit einem Innendurchmesser von 34mm angeordnet. Der Füllgrad des Gehäuses betrug ca. 41%. Die Blutprobe wurde über eine Dauer von maximal 3h mit einem Volumenstrom von 50ml/min zirkuliert, was einer linearen Fließgeschwindigkeit von 9,4 cm/min entspricht. Die Verweilzeit des Blutes im Gehäuse betrug ca. 2,5 min. Analog zu den unter Figur 1 dargestellten Untersuchungen wirrl deutlich, dass eine ausgeprägte Reduktion von Leukozyten nur bei Strömung entlang der äußeren Oberfläche erreicht wird. Obwohl die innere Oberfläche ähnlich groß ist wie die äußere Oberfläche führt eine Innendurchströmung nur zu einer geringen Reduzierung von ca 15% der Ausgangsmenge. Eine Innen- und Außenanströmung bringt keinen Vorteil gegenüber einer alleinigen Außenanströmung. Die Strömung entlang der äußeren Oberfläche parallel zum Faden bewirkt eine Reduktion der in der Blutprobe enthaltenen Leukozyten um 80%.

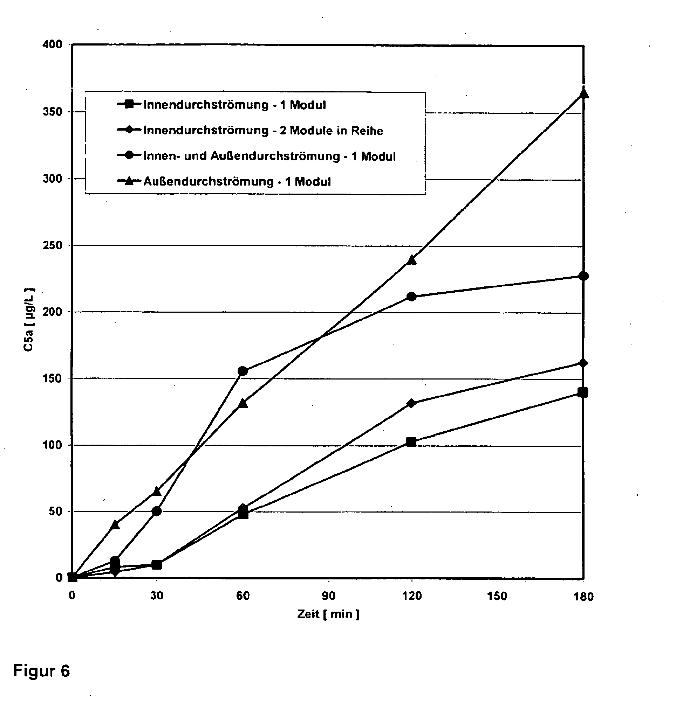

Die Bildung des Komplementaktivierungsproduktes C5a wurde unter gleichen Versuchsbedingungen und mit der gleichen Hohlfasermembran wie unter Figur 5 dargestellt untersucht. Die Ergebnisse sind in Figur 6 gezeigt. Ähnlich wie bei Figur 5 ist auch hier ein deutlicher Unterschied zwischen Innen- und Außenströmung zu beobachten. Trotz nahezu gleich großer Kontaktflächen, ist die C5a Bildung bei Außenströmung um das ca. 2,5fache höher als bei Innenströmung. Bei Außenströmung ist nach 3h eine C5a Konzentration von 365 µg/l in der Blutprobe vorhanden. Das entspricht 161 µg in der Blutprobe, bzw. von 109 µg C5a pro m² Fadenoberfläche.

### Beispiel 4

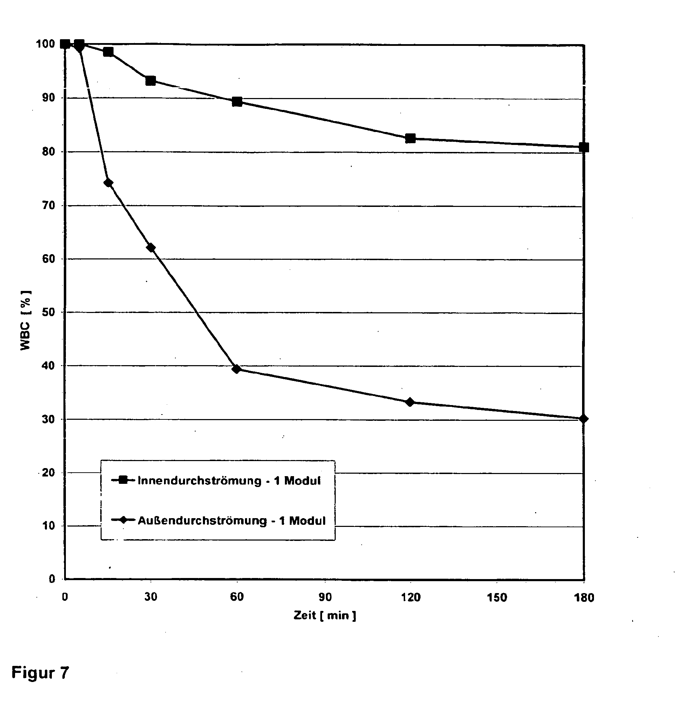

Eine Reduktion der Leukozyten unter Verwendung von Hohlfäden aus Polyethersulfon ist, wie in Figur 7 dargestellt, ebenfalls möglich. Verwendet wurden hier Hohlfäden mit einem Außendurchmesser von 300µm, einer effektiven Länge von 275mm, einer inneren Oberfläche von 1,6 m² und einer äußeren Oberfläche von 2,2 m² in einem Modul mit einem Innendurchmesser von 38,5 mm, bestückt mit 8500 Fäden, d.h. mit einem Füllgrad von ca. 52%. 381 ml Humanblut wurden mit einem Volumenstrom von 50 ml/min durch den Modul zirkuliert. Die lineare Fließgeschwindigkeit lag bei ca. 9 cm/min, die Verweilzeit bei 3,1min. Auch hier zeigt sich, dass Hohlfäden aus Polyethersulfon (PES) bei einer Außenumströmung eine Reduzierung der Leukozytenanzahl um etwa 70% bewirken. Unter den geforderten Bedingungen zur Ermittlung des C5a Gehaltes in Relation zur äußeren Fadenoberfläche wurde für die Hohlfäden aus Polyethersulfon eine flächenbezogene C5a Bildung von 30 µg/m² ermittelt.

### Beispiel 5

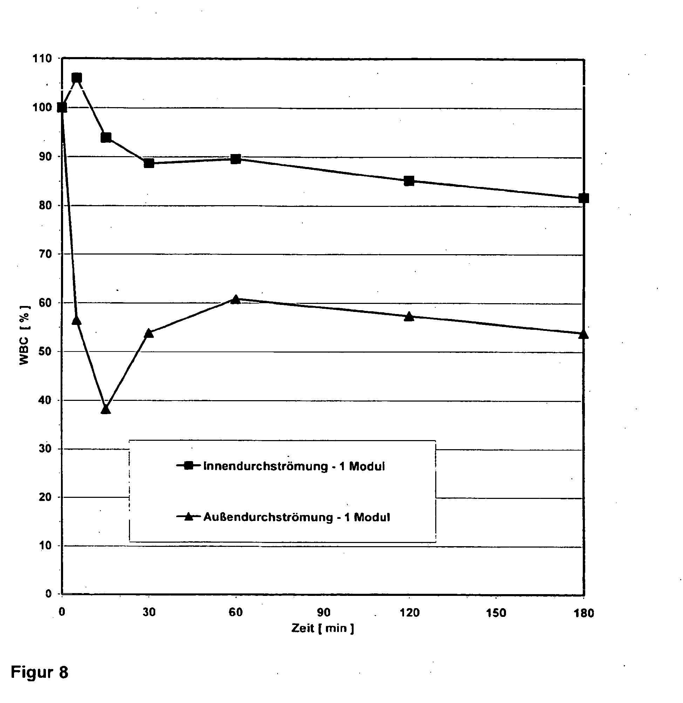

In Beispiel 5 wurden neben Hohlfäden aus Polyethersulfon noch zusätzlich Fäden aus PET für die Durchführung des erfindungsgemäßen Verfahrens verwendet. Figur 8 zeigt die zeitabhängige relative prozentuale Reduktion der Leukozyten (WBC - White Blood Cells) in einer Blutprobe behandelt mit dem erfindungsgemäßen Verfahren. Verwendet wurden hierfür Hohlfäden aus Polyethersulfon (PES) kombiniert mit Fäden aus Polyethylenterephtalat (PET) mit einer inneren Oberfläche von 1,31 m² und einer äußeren Oberfläche von 1,57 m². 8000 Fäden mit einem Fadendurchmesser von 260µm und einer Länge von 240mm wurden dafür in einem zylindrischen Gehäuse mit einem Innendurchmesser von 34mm angeordnet. Der Füllgrad des Gehäuses betrug ca. 47%. Als Blutprobe wurde 470 ml Humanblut verwendet, das über eine Dauer von maximal 3h mit einem Volumenstrom von 50ml/min zirkuliert wurde, was einer linearen Fließgeschwindigkeit von 10 cm/min entspricht. Die Verweilzeit des Blutes im Gehäuse betrug 2,3min. Hier zeigt sich, dass Hohlfäden aus Polyethersulfon in Kombination mit Polyethylenterephtalat -Fäden bei einer Außenumströmung eine Reduzierung der Leukozytenanzahl um etwa 50% bewirken. Eine Innendurchströmung führt nur zu einer geringen Reduzierung um ca. 18% der Ausgangsmenge.

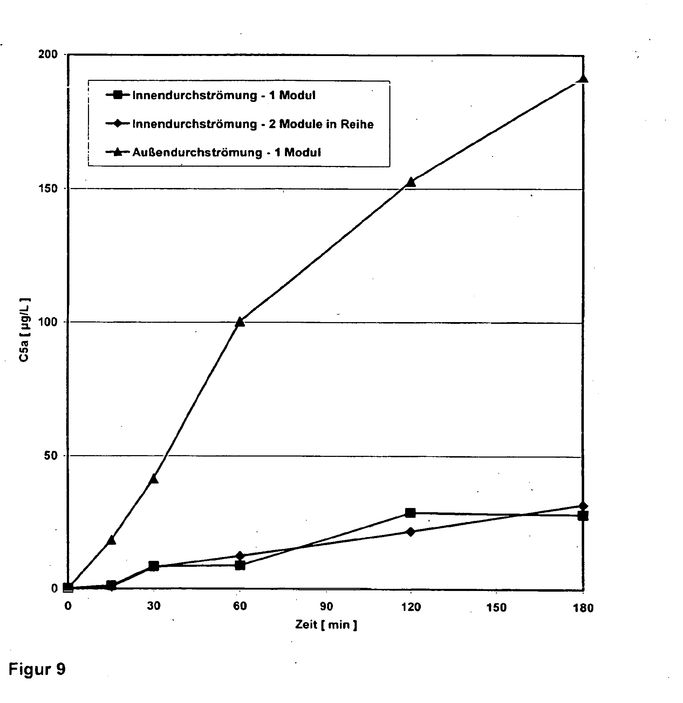

Analog zu den in Figur 8 dargestellten Ergebnissen, gibt es abhängig von der Durchströmung auch große Unterschiede bei der C5a Bildung. Die in Figur 9 dargestellten Ergebnisse wurden mit der gleichen Membran wie in Figur 8 beschrieben und unter den geforderten Bedingungen zur Ermittlung des C5a Gehaltes in Relation zur Fadenoberfläche ermittelt. Bei Innenströmung wurde eine C5a Konzentration von lediglich 30 µg/l ermittelt, bei Außenströmung eine C5a Konzentration von 192 µg/l. Letzteres entspricht einer C5a Bildung von 90 µg in der behandelten Blutprobe, bzw. von 58 µg pro m² Fadenoberfläche.

## Patentansprüche

1. Verfahren zur Reduzierung der Leukozytenzahl in Blut durch eine in vitro Behandlung des Blutes in einer Anordnung einer Vielzahl von Fäden auf Basis organischer Polymere, wobei die Vielzahl von Fäden in einem Gehäuse mit einer Einlasseinrichtung und einer Auslasseinrichtung gehalten werden, das Blut über die Einlasseinrichtung dem Gehäuse zugeführt wird, anschließend das Gehäuse durchströmt, wobei das Blut die Fäden an ihrer äußeren Oberfläche umströmt, und schließlich über die Auslasseinrichtung das Gehäuse wieder verlässt, **dadurch gekennzeichnet, dass** Fäden eingesetzt werden, die bei Umströmung ihrer äußeren Oberfläche mit Blut eine Bildung des Komplementaktivierungsproduktes C5a in einer Konzentration von mindestens 10 µg pro m² Fadenoberfläche bewirken und dass die Anordnung der Fäden einen hohen Grad an Ordnung aufweist, wobei unter einem hohen Grad an Ordnung zu verstehen ist, dass ein hoher Anteil der Fäden entlang ihrer Erstreckungsrichtung nebeneinander angeordnet sind.

2. Verfahren nach Anspruch 1, **dadurch gekennzeichnet, dass** die Fäden eine Bildung des Komplementaktivierungsproduktes C5a in einer Konzentration von mindestens 75 µg pro m² Fadenoberfläche Blut bewirken.

3. Verfahren nach Anspruch 1 oder 2, **dadurch gekennzeichnet, dass** die Fäden auf Basis organischer Polymere Hohlfäden sind und das Blut die Hohlfäden an ihrer äußeren Oberfläche umströmt.

4. Verfahren nach einem oder mehreren der Ansprüche 1 bis 3 **dadurch gekennzeichnet, dass** die Anzahl der Fäden auf Basis organischer Polymere im Bereich von 2.000 bis 20.000 Fäden liegt.

5. Verfahren nach einem oder mehreren der Ansprüche 1 bis 4 **dadurch gekennzeichnet, dass** der Fadendurchmesser der Fäden auf Basis organischer Polymere zwischen 0,05 mm und 2 mm liegt.

6. Verfahren nach einem oder mehreren der Ansprüche 1 bis 5 **dadurch gekennzeichnet, dass** die spezifische Oberfläche der Anordnung der Fäden auf Basis organischer Polymere zwischen 0,1 und 100 cm² pro ml zu behandelnden Blutes liegt.

7. Verfahren nach einem oder mehreren der Ansprüche 1 bis 6 **dadurch gekennzeichnet, dass** die Fäden auf Basis organischer Polymere aus regenerierter Zellulose, Zelluloseazetat oder mit Benzylgruppen modifizierter Zellulose (Benzylzellulose) bestehen.

8. Verfahren nach Anspruch 1 **dadurch gekennzeichnet, dass** die Fäden auf Basis organischer Polymere im wesentlichen aus Polyethersulfon oder Polysulfon bestehen.

9. Verfahren nach Anspruch 7 oder 8 **dadurch gekennzeichnet, dass** die Anordnung einer Vielzahl von Fäden zusätzlich auch Fäden aus Polyethylenterephthalat enthält.

10. Verfahren nach einem oder mehreren der Ansprüche 1 bis 9, **dadurch gekennzeichnet, dass** der Füllgrad der Fäden im Gehäuse im Bereich von 10% bis 70% liegt.

11. Verfahren nach einem oder mehreren der Ansprüche 1 bis 10, **dadurch gekennzeichnet, dass** die Verweilzeit des Blutes im Gehäuse 0,5 min bis 5 min beträgt.

12. Verfahren nach einem oder mehreren der Ansprüche 1 bis 11 **dadurch gekennzeichnet, dass** die lineare Fließgeschwindigkeit, mit der das Blut durch das Gehäuse strömt, zwischen 5 und 30 cm/min beträgt.

13. Verfahren nach einem oder mehreren der Ansprüche 1 bis 12 **dadurch gekennzeichnet, dass** die Fäden auf Basis organischer Polymere als Fadenbündel mit im wesentlichen parallelen Fäden vorliegen.

14. Verfahren nach einem oder mehreren der Ansprüche 1 bis 13 **dadurch gekennzeichnet, dass** die Fäden auf Basis organischer Polymere in einer oder mehrerer Lagen vorliegen und dass die Fäden innerhalb einer Lage im wesentlichen parallel liegen.

15. Vorrichtung zur Reduzierung von Leukozyten aus Blut umfassend eine Vielzahl von Fäden auf Basis organischer Polymere in einem Gehäuse mit einer Einlasseinrichtung und einer Auslasseinrichtung, wobei die Fäden mit zumindest einem ihrer Enden so in eine mit der Gehäuseinnenseite verbundenen Vergussmasse eingebettet sind, dass um die Fäden herum ein von Blut durchströmbarer Außenraum ausgebildet ist, **dadurch gekennzeichnet, dass** die Anordnung der Fäden einen hohen Grad an Ordnung aufweist, wobei unter einem hohen Grad an Ordnung zu verstehen ist, dass ein hoher Anteil der Fäden entlang ihrer Erstreckungsrichtung nebeneinander angeordnet sind, und dass die Fäden auf Basis organischer Polymere eine Bildung des Komplementaktivierungsproduktes C5a in einer Konzentration von mindestens 10 µg pro m² Fadenoberfläche bewirken.

## Claims

1. Method for reducing the number of leukocytes in blood through an in vitro treatment of the blood in an arrangement of a plurality of fibers based on organic polymers, wherein the plurality of fibers is contained in a housing with an inlet arrangement and an outlet arrangement, the blood is channeled via the inlet arrangement into the housing, then flows through the housing, wherein the blood flows around the fibers on their external surface, and finally exits the housing via the outlet arrangement, **characterized in that** fibers are used that cause a generation of the complement activation product C5a in a concentration of at least 10 µg per m² of fiber surface when blood flows around the external surface of said fibers, and that the arrangement of the fibers has a high degree of order, whereby a high degree of order is understood to mean that a high proportion of the fibers are arranged side by side along their direction of extension.

2. Method according to Claim 1, **characterized in that** the fibers cause a generation of the complement activation product C5a in a concentration of at least 75 µg per m² of fiber surface.

3. Method according to Claim 1 or 2, **characterized in that** the fibers based on organic polymers are hollow fibers and the blood flows around them along their external surface.

4. Method according to one or more of Claims 1 to 3, **characterized in that** the number of the fibers based on organic polymers lies in the range of 2000 to 20,000 fibers.

5. Method according to one or more of Claims 1 to 4, **characterized in that** the diameter of the fibers based on organic polymers lies between 0.05 mm and 2 mm.

6. Method according to one or more of Claims 1 to 5, **characterized in that** the specific surface area of the arrangement of the fibers based on organic polymers lies between 0.1 and 100 cm² per ml of blood to be treated.

7. Method according to one or more of Claims 1 to 6, **characterized in that** the fibers based on organic polymers consist of regenerated cellulose, cellulose acetate, or cellulose modified with benzyl groups (benzyl cellulose).

8. Method according to Claim 1, **characterized in that** the fibers based on organic polymers consist essentially of polyether sulfone or polysulfone.

9. Method according to Claim 7 or 8, **characterized in that** the arrangement of a plurality of fibers also contains fibers made of polyethylene terephthalate.

10. Method according to one or more of Claims 1 to 9, **characterized in that** the fill ratio of the fibers in the housing lies in the range from 10% to 70%.

11. Method according to one or more of Claims 1 to 10, **characterized in that** the retention time of the blood in the housing is from 0.5 min to 5 min.

12. Method according to one or more of Claims 1 to 11, **characterized in that** the linear flow rate, at which the blood flows through the housing, is between 5 and 30 cm/min.

13. Method according to one or more of Claims 1 to 12, **characterized in that** the fibers based on organic polymers are present as a fiber bundle with essentially parallel fibers.

14. Method according to one or more of Claims 1 to 13, **characterized in that** the fibers based on organic polymers are present in one or more layers, and that the fibers within each layer lie essentially parallel.

15. Device for reducing the leukocytes from blood comprising a plurality of fibers based on organic polymers in a housing with an inlet arrangement and an outlet arrangement, wherein at least one of the ends of the fibers is embedded in a sealing compound connected to the interior side of the housing, so that an outer space through which the blood can flow is formed around the fibers, **characterized in that** the arrangement of the fibers has a high degree of order, whereby a high degree of order is understood to mean that a high proportion of the fibers are arranged side by side along their direction of extension, and that the fibers based on organic polymers cause a generation of the complement activation product C5a in a concentration of at least 10 µg per m² of fiber surface.

## Revendications

1. Procédé permettant de réduire le nombre de leucocytes dans du sang, au moyen d'un traitement in vitro du sang dans un ensemble d'un grand nombre de filaments à base de polymère organique, dans lequel les nombreux filaments sont maintenus en place dans un boîtier pourvu d'un dispositif d'entrée et d'un dispositif de sortie, et le sang pénètre dans le boîtier en passant par le dispositif d'entrée, puis traverse le boîtier, en passant autour des filaments et en coulant sur les surfaces externes de ceux-ci, et enfin quitte le boîtier en passant par le dispositif de sortie, **caractérisé en ce que** l'on utilise des filaments qui, lorsque du sang coule sur leur surface externe, entraînent la formation du produit C5a d'activation du complément, en une concentration d'au moins 10 microgrammes par mètre carré de surface des filaments, et **en ce que** les filaments sont disposés avec un haut degré d'ordre, l'expression "haut degré d'ordre" signifiant qu'une grande proportion des filaments sont disposés les uns à côté des autres dans leur direction axiale.

2. Procédé selon la revendication 1, **caractérisé en ce que** les filaments entraînent la formation du produit C5a d'activation du complément en une concentration d'au moins 75 microgrammes par mètre carré de surface de filaments touchée par le sang.

3. Procédé selon la revendication 1 ou 2, **caractérisé en ce que** les filaments à base de polymère organique sont des fibres creuses et **en ce que** le sang passe autour des fibres creuses en coulant sur les surfaces externes de celles-ci.

4. Procédé selon l'une ou plusieurs des revendications 1 à 3, **caractérisé en ce que** les filaments à base de polymère organique sont au nombre de 2 000 à 20 000.

5. Procédé selon l'une ou plusieurs des revendications 1 à 4, **caractérisé en ce que** les filaments à base de polymère organique ont de 0,05 à 2 mm de diamètre.

6. Procédé selon l'une ou plusieurs des revendications 1 à 5, **caractérisé en ce que** la surface spécifique de l'ensemble de fila-ments à base de polymère organique vaut de 0,1 à 100 cm², par milli-litre de sang à traiter.

7. Procédé selon l'une ou plusieurs des revendications 1 à 6, **caractérisé en ce que** les filaments à base de polymère organique sont faits de cellulose régénérée, d'acétate de cellulose, ou de benzyl-cellulose (cellulose modifiée avec des groupes benzyle).

8. Procédé selon la revendication 1, **caractérisé en ce que** les filaments à base de polymère organique sont essentiellement faits de poly(éther sulfone) ou de polysulfone.

9. Procédé selon la revendication 7 ou 8, **caractérisé en ce que** l'ensemble d'un grand nombre de filaments comprends en outre des filaments faits de poly(éthylène téréphtalate).

10. Procédé selon l'une ou plusieurs des revendications 1 à 9, **caractérisé en ce que** le taux de remplissage du boîtier par les filaments vaut de 10 à 70 %.

11. Procédé selon l'une ou plusieurs des revendications 1 à 10, **caractérisé en ce que** le temps de passage du sang dans le boîtier vaut de 0,5 à 5 minutes.

12. Procédé selon l'une ou plusieurs des revendications 1 à 11, **caractérisé en ce que** le sang traverse le boîtier avec une vitesse d'écoulement linéaire de 5 à 30 cm/min.

13. Procédé selon l'une ou plusieurs des revendications 1 à 12, **caractérisé en ce que** les filaments à base de polymère organique sont disposés en un faisceau de filaments sensiblement parallèles.

14. Procédé selon l'une ou plusieurs des revendications 1 à 13, **caractérisé en ce que** les filaments à base de polymère organique sont disposés en une ou plusieurs couches et **en ce que** les filaments, au sein d'une couche, sont sensiblement parallèles.

15. Appareil conçu pour réduire le nombre de leucocytes dans du sang, comportant un grand nombre de filaments à base de polymère organique, placés dans un boîtier pourvu d'un dispositif d'entrée et d'un dispositif de sortie, dans lequel les filaments ont au moins l'une de leurs extrémités noyée dans une masse de coulée attachée à la paroi interne du boîtier, de telle manière qu'autour des filaments est laissé un espace extérieur par lequel du sang peut passer, **caractérisé en ce que** les filaments sont disposés avec un haut degré d'ordre, l'expression "haut degré d'ordre" signifiant qu'une grande proportion des filaments sont disposés les uns à côté des autres dans leur direction axiale, et **en ce que** les filaments à base de polymère organique entraînent la formation du produit C5a d'activation du complément, en une concentration d'au moins 10 microgrammes par mètre carré de surface des filaments.
